# EUROPEAN PATENT APPLICATION

(11) **EP 3 613 337 A1**
(43) Date of publication of application: **26.02.2020**
(21) Application number: 18190236.2
(22) Date of filing: 22.08.2018
(51) Int. Cl.: A61B 5/00, A61B 5/024, A61B 5/1455

(54) **AN APPARATUS, METHOD AND COMPUTER PROGRAM FOR DETERMINING A BIOMETRIC PARAMETER**

(71) Applicant: Nokia Technologies Oy, 02610 Espoo (FI)
(72) Inventor: Bitauld, David, Cambridge, Cambridgeshire CB2 8DL (GB)
(74) Representative: Swindell & Pearson Limited

(57) **Abstract**

Examples of the disclosure relate to an apparatus, method and computer program for determining biometric parameters. The apparatus comprises means for: obtaining a signal from a broadband detector in response to light that has been propagated through a subject being incident on the broadband detector. The signal that is obtained comprises at least a first component and a second component. The first component is provided in response to a first spectral and spatial distribution of light being incident on the broadband detector and the second component is provided in response to at least one other spectral and spatial distribution of light being incident on the broadband detector. An emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to a biometric parameter. The apparatus also has means for processing the first component and the second component of the obtained signal to enable the biometric parameter of the subject to be determined.

## Description

### TECHNOLOGICAL FIELD

Examples of the present disclosure relate to an apparatus, method and computer program for determining a biometric parameter. Some relate to an apparatus, method and computer program for determining a biometric parameter using photoplethsmography.

### BACKGROUND

Apparatus for measuring biometric parameters using methods such as photoplethsmography are known. In such apparatus it is necessary to take into account factors that can affect the accuracy of the measurements such as motion artefacts.

### BRIEF SUMMARY

According to various, but not necessarily all, examples of the disclosure there is provided an apparatus comprising means for: obtaining a signal from a broadband detector in response to light that has been propagated through a subject being incident on the broadband detector, wherein the signal comprises at least a first component and a second component where the first component is provided in response to a first spectral and spatial distribution of light being incident on the broadband detector and the second component is provided in response to at least one other spectral and spatial distribution of light being incident on the broadband detector and wherein an emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to a biometric parameter; and processing the first component and the second component of the obtained signal to enable the biometric parameter of the subject to be determined.

Controlling the emitters so that the first spectral and spatial distribution of light and the at least one other spectral and spatial distribution of light have a similar dependency to motion artefacts but a different dependency to the biometric parameter may comprise selecting one or more wavelengths of light to use in the spectral distributions.

Controlling the emitters so that the first spectral and spatial distribution of light and the at least one other spectral and spatial distribution light have a similar dependency to motion artefacts but a different dependency to the biometric parameter may comprise positioning the emitters relative to the broadband detector.

The signal may also comprise at least a second component and a third component wherein the second component may be provided in response to a second spectral and spatial distribution of light being incident on the broadband detector and the third component may be provided in response to a third spectral and spatial distribution of light being incident on the broadband detector.

The emission of the respective spectral and spatial distributions of light may be controlled so that the respective spectral and spatial distributions of light are incident on the broadband detector at different times.

The first spectral and spatial distribution of light may comprise green light. The at least one other spectral and spatial distribution of light may comprise at least one of; red light, blue light.

The biometric parameter may comprise the heart rate of the subject.

Processing the respective components of the signal to determine a biometric parameter of the subject may comprise filtering at least the respective components of the signal to separate a common mode signal from a differential signal. The differential signal may comprise information about the biometric parameter.

The respective components of the signal may be filtered at a frequency corresponding to a frequency at which the spectral and spatial distributions of light are alternated.

The emitter of the first spectral and spatial distribution of light and the emitter of the at least one other spectral and spatial distribution of light may be controlled so that a similar power level is incident on the broadband detector for both of the spectral and spatial distributions of light.

According to various, but not necessarily all, examples of the disclosure there is provided an apparatus comprising: processing circuitry; and memory circuitry including computer program code, the memory circuitry and the computer program code configured to, with the processing circuitry, cause the apparatus to: obtain a signal from a broadband detector in response to light that has been propagated through a subject being incident on the broadband detector, wherein the signal comprises at least a first component and a second component where the first component is provided in response to a first spectral and spatial distribution of light being incident on the broadband detector and the second component is provided in response to at least one other spectral and spatial distribution of light being incident on the broadband detector and wherein an emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to a biometric parameter; and process the first component and the second component of the obtained signal to enable the biometric parameter of the subject to be determined.

According to various but not necessarily all, example of the disclosure, there is provided a method comprising: obtaining a signal from a broadband detector in response to light that has been propagated through a subject being incident on the broadband detector, wherein the signal comprises at least a first component and a second component where the first component is provided in response to a first spectral and spatial distribution of light being incident on the broadband detector and the second component is provided in response to at least one other spectral and spatial distribution of light being incident on the broadband detector and wherein an emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to a biometric parameter; and processing the first component and the second component of the obtained signal to enable the biometric parameter of the subject to be determined.

According to various but not necessarily all, example of the disclosure, there is provided a computer program comprising computer program instructions that, when executed by processing circuitry, cause: obtaining a signal from a broadband detector in response to light that has been propagated through a subject being incident on the broadband detector, wherein the signal comprises at least a first component and a second component where the first component is provided in response to a first spectral and spatial distribution of light being incident on the broadband detector and the second component is provided in response to at least one other spectral and spatial distribution of light being incident on the broadband detector and wherein an emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to a biometric parameter; and processing the first component and the second component of the obtained signal to enable the biometric parameter of the subject to be determined.

According to various but not necessarily all, example of the disclosure, there is provided a physical entity comprising a computer program as described above.

According to various but not necessarily all, example of the disclosure, there is provided a system comprising: at least a first emitter configured to emit light of a first spectral and spatial distribution; at least a second emitter configured to emit light of a second spectral and spatial distribution; at least one broadband detector configured to detect at least the first spectral and spatial distribution of light and the second spectral and spatial distribution of light after the respective spectral and spatial distributions of light have propagated through a subject such that the detected signals enable a biometric parameter of the subject to be determined; and control means configured to control the emission of the first spectral and spatial distribution of light and the second spectral and spatial distribution of light so that they are incident on the broadband detector at different times; wherein the first emitter and the second emitter are controlled so that the first spectral and spatial distribution of light and the second spectral and spatial distribution of light have a similar dependency to motion artefacts but a different dependency to the biometric parameter.

Controlling the emitters so that the first spectral and spatial distribution of light and the second spectral and spatial distribution light have a similar dependency to motion artefacts but a different dependency to the biometric parameter may comprise positioning the emitters relative to the broadband detector so that the first spectral and spatial distribution of light and the second spectral and spatial distribution of light probe a similar depth of the subject.

Controlling the emitters so that the first spectral and spatial distribution of light and the second spectral and spatial distribution light have a similar dependency to motion artefacts but a different dependency to the biometric parameter may comprise selecting one or more wavelengths of light to use in the spectral distributions.

The system may comprise at least a third emitter configured to emit a third spectral and spatial distribution of light.

The first spectral and spatial distribution of light may comprise green light and the second spectral and spatial distribution of light and/or the third spectral and spatial distribution of light comprises light may comprise at least one of; red light, blue light.

The biometric parameter may comprise the heart rate of the subject.

The emitter of the first spectral and spatial distribution of light may be provided at a different distance from the broadband detector to the emitter of the second spectral and spatial distribution of light and/or the emitter of the third spectral and spatial distribution of light.

The emitter of the first spectral and spatial distribution of light and the emitter of the second spectral and spatial distribution of light and/or the emitter of the third spectral and spatial distribution of light may be controlled so that a similar power level is incident on the broadband detector for each of the spectral and spatial distributions of light.

### BRIEF DESCRIPTION

Some example embodiments will now be described with reference to the accompanying drawings in which:
Fig. 1 shows an apparatus;
Fig. 2 shows a system;
Figs. 3A and 3B show a system and signals that may be obtained using the system;
Fig. 4 shows how different spectral distributions of light may be propagated through a subject;
Fig. 5 shows an example configuration of a broadband detector and a plurality of emitters;
Fig. 6 shows another example configuration of a plurality broadband detectors and a plurality of emitters;
Fig. 7 shows a filter arrangement;
Fig. 8 shows example signals;
Fig. 9 shows example signals; and
Fig. 10 shows example method.

### DETAILED DESCRIPTION

Examples of the disclosure relate to an apparatus comprising means for obtaining a signal from a broadband detector in response to light that has been propagated through a subject being incident on the broadband detector and processing the components of the obtained signal to enable a biometric parameter of the subject to be determined. The signal that is obtained comprises at least a first component and a second component where the first component is provided in response to a first spectral and spatial distribution of light being incident on the broadband detector and the second component is provided in response to at least one other spectral and spatial distribution of light being incident on the broadband detector. An emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to the biometric parameter.

The apparatus may be for monitoring biometric parameters. In some examples the apparatus may be for monitoring biometric parameters using photoplethsmography.

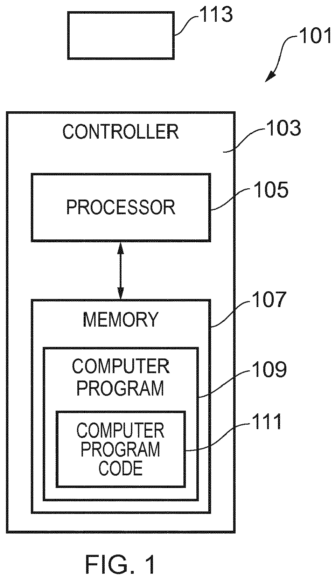

Fig.1 schematically illustrates an apparatus 101 according to examples of the disclosure. In the example of Fig. 1 the apparatus 101 comprises a controller 103. In the example of Fig. 1 the implementation of the controller 103 may be as controller circuitry. In some examples the controller 103 may be implemented in hardware alone, have certain aspects in software including firmware alone or can be a combination of hardware and software (including firmware).

As illustrated in Fig. 1 the controller 103 may be implemented using instructions that enable hardware functionality, for example, by using executable instructions of a computer program 109 in a general-purpose or special-purpose processor 105 that may be stored on a computer readable storage medium (disk, memory etc) to be executed by such a processor 105.

The processor 105 is configured to read from and write to the memory 107. The processor 105 may also comprise an output interface via which data and/or commands are output by the processor 105 and an input interface via which data and/or commands are input to the processor 105.

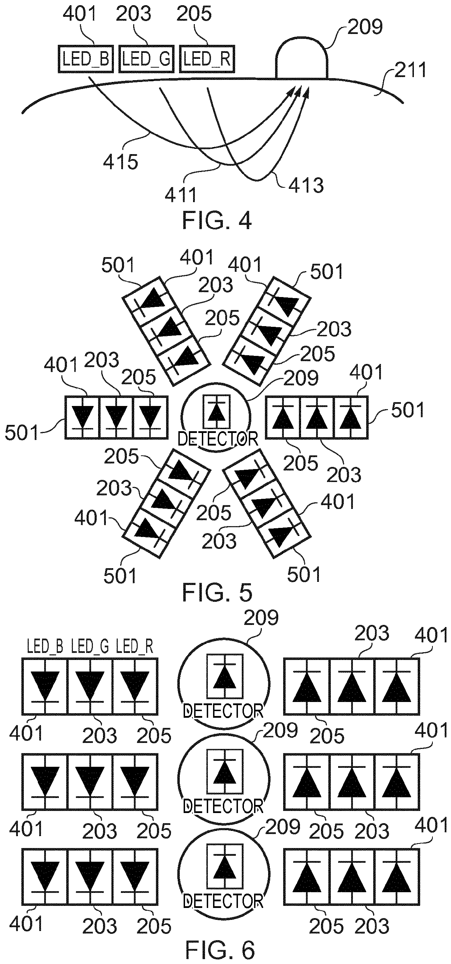

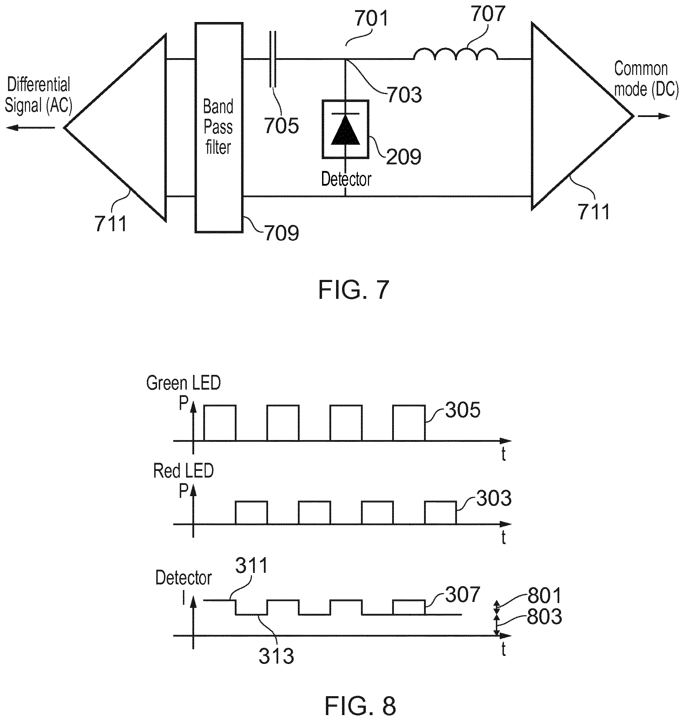

The memory 107 is configured to store a computer program 109 comprising computer program instructions (computer program code 111) that controls the operation of the apparatus 101 when loaded into the processor 105. The computer program instructions, of the computer program 109, provide the logic and routines that enables the apparatus 101 to perform the methods illustrated in Figs. 4 and 8. The processor 105 by reading the memory 107 is able to load and execute the computer program 109.

The apparatus 101 therefore comprises: at least one processor 105; and at least one memory 107 including computer program code 111, the at least one memory 107 and the computer program code 111 configured to, with the at least one processor 105, cause the apparatus 101 at least to perform: obtaining 1001 a signal from a broadband detector in response to light that has been propagated through a subject 211 being incident on the broadband detector 209, wherein the signal comprises at least a first component and a second component where the first component is provided in response to a first spectral and spatial distribution of light being incident on the broadband detector 209 and the second component is provided in response to at least one other spectral and spatial distribution of light being incident on the broadband detector 209 and wherein an emitter 203 of the first spectral and spatial distribution of light and an emitter 205 of the at least one other spectral and spatial distribution of light are controlled so that the first spectral and spatial distribution of light and the at least one other spectral and spatial distribution light have a similar dependency to motion artefacts but a different dependency to a biometric parameter; and processing 1003 the first component and the second component of the obtained signal to enable the biometric parameter of the subject 211 to be determined.

As illustrated in Fig.1 the computer program 109 may arrive at the apparatus 101 via any suitable delivery mechanism 113. The delivery mechanism 113 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 109. The delivery mechanism may be a signal configured to reliably transfer the computer program 109. The apparatus 101 may propagate or transmit the computer program 109 as a computer data signal. In some examples the computer program 109 may be transmitted to the apparatus 101 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 109 comprises computer program instructions for causing an apparatus 101 to perform at least the following: obtaining 1001 a signal from a broadband detector in response to light that has been propagated through a subject 211 being incident on the broadband detector 209, wherein the signal comprises at least a first component and a second component where the first component is provided in response to a first spectral and spatial distribution of light being incident on the broadband detector 209 and the second component is provided in response to at least one other spectral and spatial distribution of light being incident on the broadband detector 209 and wherein an emitter 203 of the first spectral and spatial distribution of light and an emitter 205 of the at least one other spectral and spatial distribution of light are controlled so that the first spectral and spatial distribution of light and the at least one other spectral and spatial distribution light have a similar dependency to motion artefacts but a different dependency to a biometric parameter; and processing 1003 the first component and the second component of the obtained signal to enable the biometric parameter of the subject 211 to be determined.

The computer program instructions may be comprised in a computer program 109, a non-transitory computer readable medium, a computer program product, a machine readable medium. In some but not necessarily all examples, the computer program instructions may be distributed over more than one computer program 109.

Although the memory 107 is illustrated as a single component/circuitry it may be implemented as one or more separate components/circuitry some or all of which may be integrated/removable and/or may provide permanent/semi-permanent/ dynamic/cached storage.

Although the processor 105 is illustrated as a single component/circuitry it may be implemented as one or more separate components/circuitry some or all of which may be integrated/removable. The processor 105 may be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software may not be present when it is not needed for operation.
This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

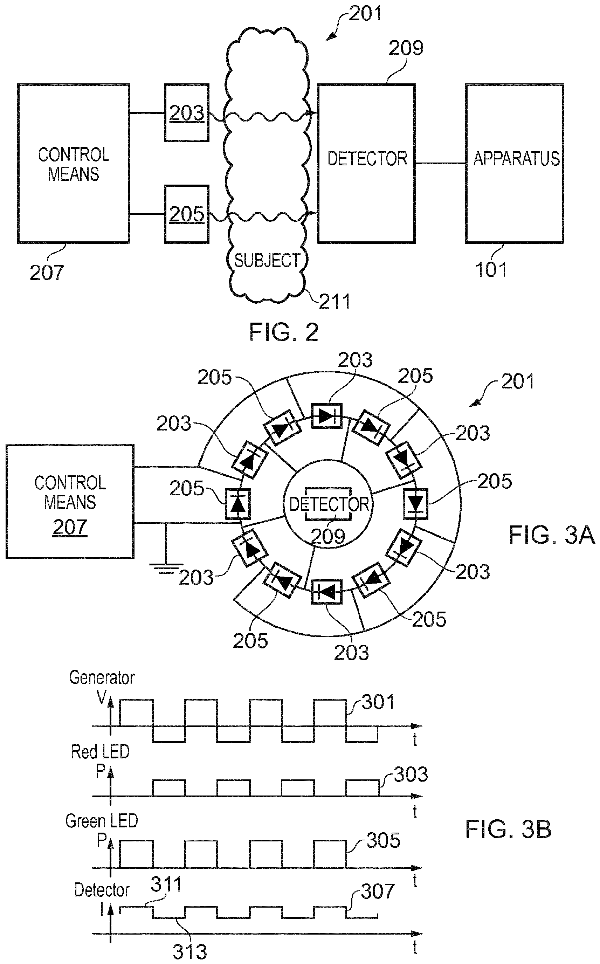

Fig. 2 schematically illustrates a system 201 that may be used to implement some examples of the disclosure. In the examples of the disclosure the system 201 comprises at least a first emitter 203 and a second emitter 205, control means 207, and at least one broadband detector 209. The broadband detector 209 may be coupled to an apparatus 101 such as the apparatus 101 shown in Fig. 1.

In some examples the system 201, or at least some components of the system 201 could be provided in a wearable device. The wearable device could comprise a watch type device which could be attached to the wrist of a subject 211, a chest strap which could be attached to the torso of a subject 211 or any other suitable type of device. The wearable device could be configured so that when the device is attached to the subject 211 the emitters 203, 205 and the detector 209 are provided adjacent to, or in close proximity to, the skin of the subject 211. This enables at least some of the light from the emitters 203 to be propagated through the subject 211 and incident on the broadband detector 209.

The emitters 203, 205 may comprise any means which may be configured to emit a spectral and spatial distribution of light. The emitters 203, 205 may comprise one or more light sources. The light sources could comprise light emitting diodes (LEDs) or any other suitable means.

The light sources within the emitters 203, 205 are selected so as to control the spectral distribution of light emitted by the emitters 203, 205. The light sources may be selected so that the first emitter 203 is configured to emit light having a first spectral distribution and the second emitter 205 is configured to emit light having a second, different spectral distribution.

The different spectral distributions could comprise different wavelengths of light. In some examples the first spectral distribution could comprise green light. In some examples the first spectral distribution could have a wavelength between 520nm and 560nm. The green light used for the first spectral distribution may be selected as the absorption of the green light within a subject 211 gives an indication of the volume of blood within the subject 211.

The second spectral distribution could comprise at least one of red light and blue light. In some examples the second spectral distribution could comprise light that is not green. In some examples the second spectral distribution could comprise white light. In some examples the second spectral distribution could comprise light having wavelengths between 635nm and 700nm and/or between 450nm and 490nm. The wavelengths of light used in the second spectral distribution may be selected so that the light has a similar dependency to motion artefacts within the subject 211 as the first spectral distribution of light but has a different dependency to the biometric parameter that is to be measured. The similarity between the dependencies of the light to the motion artefacts may be such that the motion artefacts can be removed from signals obtained by the broadband detector 209 by a filter or other suitable means.

In some examples the system 201 is configured so that the emitters 203, 205 are positioned relative to the broadband detector 209 so that the light from the respective emitters 203, 205 has a similar dependency to motion artefacts within the subject 211 but has a different dependency to the biometric parameter. In some examples the emitters 203, 205 are positioned relative to the broadband detector 209 so that the light from each of the respective emitters 203, 205 has a similar dependency to motion artefacts within the subject 211 but has a different dependency to the biometric parameter. The similar dependency to the motion artefacts may be obtained by positioning the emitters 203, 205 relative to the broadband detector 209 so that the respective distributions of light probe a similar depth of the subject 211. In some examples the emitters 203, 205 may be positioned relative to the broadband detector 209 so that the path lengths of the distributions of light through the subject 211 are such that a similar amount of light is absorbed for each of the distributions of light.

In some example systems 201 the emitters 203, 205 may be positioned close to each other so that the different spectral and spatial distributions of light are emitted through similar parts of the subject 211. In some examples the emitters 203, 205 may be positioned so that at least some of the different spectral and spatial distributions of light are propagated through the same part of the subject 211. This may help to ensure that the light from each of the emitters 203, 205 has a similar dependency to motion artefacts within the subject 211.

In the example of Fig. 2 the system comprises two emitters 203, 205. It is to be appreciated that in other examples of the disclosure the system 201 could comprise more than two emitters 203, 205. For example the system 201 could comprise at least a third emitter which could be configured to provide a third spectral and spatial distribution of light to be incident on the broadband detector 209.

In the example of Fig. 2 the system 201 also comprises control means 207. The control means 207 may comprise any means which may be configured to control the emitters 203, 205. The control means 207 may be configured to provide control signals to the emitters 203, 205. In some examples the control means 207 could comprises a controller 103 which may comprise a processor 105 and memory 107 or any other suitable means. The control means 207 could comprise components as shown in Fig. 1.

The control means 207 may be configured to control the light that is emitted by the emitters 203, 205. In some examples the control means 207 may be configured to control the intensity of the light that is emitted. The intensity of the light that is emitted may be controlled so that a similar power level is incident on the broadband detector 209 for each of the spatial and spectral distributions of light. The power level may be similar so as to facilitate the filtering of the different components of the output signal provided by the broadband detector 209.

The control means 207 may be configured to control an illumination sequence of the emitters. The control means 207 may be configured to control an illumination sequence of the emitters 203, 205 so that the emission of light by the emitters 203, 205 is alternated. The emission of light by the emitters 203, 205 may be alternated so that the different spectral and spatial distributions of light emitted by the different emitters 203, 205 are incident on the broadband detector 209 at different times. In some examples the emission of the different spectral and spatial distributions of light may be alternated so that the maximum intensity of the different spectral and spatial distributions of light are incident on the broadband detector 209 at different times. This alternation may ensure that the different spectral and spatial distributions of light are modulated in antiphase. The emission of the different spectral and spatial distributions may be alternated at a frequency controlled by the control means 207. The frequency may be higher than the frequency of a user's heart rate or other biometric parameter that the system 201 may be measuring so that the common mode signal may be removed by filtering at a frequency corresponding to the frequency at which the emission of the spectral and spatial distributions of light are alternated.

The alternation between the different spectral and spatial distributions light could be a square wave so that if the first emitter 203 is illuminated the second emitter 205 is not illuminated and similarly if the second emitter 205 is illuminated the first emitter 203 is not illuminated. In such examples the emitters 203, 205 have only two states which is either off or on. In other examples the different emitter 203, 205 could be controlled to provide a sinusoidal output. The sinusoidal outputs may vary in intensity over time. The sinusoidal outputs for the different emitters may be in antiphase so that when one emitter 203, 205 has a maximum intensity output the other emitter has a minimum intensity output. The minimum intensity output may be zero.

The broadband detector 209 may be positioned within the system 201 so that, when the system 201 is in use, light from the each of the emitters 203, 205 is propagated through the subject 211 and is incident on the broadband detector 209.

The broadband detector 209 may comprise any means which may be configured to convert incident light into an electrical output signal. For instance, the broadband detector 209 may comprise one or more photodiodes or any other suitable means. The electrical output signal provided by the broadband detector 209 may provide an indication of the intensity of light that was incident on the broadband detector 209 as function of time. The broadband detector 209 may be configured to detect light from each of the first spectral and spatial distributions used within the system 201. This enables the same detector 209 to be used to detect each of the different spectral and spatial distributions of light so that only a single detector 209 is needed.

As the broadband detector 209 detects different spectral and spatial distributions of light the broadband detector 209 therefore provides a single output signal that comprises a plurality of different components. The different components correspond to the different spectral and spatial distributions of light that are detected. In examples of the disclosure where the emission of the different spectral and spatial distributions are alternated the different components of the signal corresponding to the different spectral and spatial distributions of light are detected by the broadband detector 209 at different times.

In the example system 201 of Fig. 2 the broadband detector 209 is configured to provide an output signal to an apparatus 101. This enables the apparatus 101 to obtain a signal comprising a plurality of different components. The apparatus 101 may be configured to process the signal and the plurality of different components within the signal to obtain a measurement of a biometric parameter of the subject 211. The biometric parameter could be the heart rate of the subject 211 or any other parameter that affects the way different wavelengths of light are absorbed by the subject 211.

In some examples the apparatus 101 could comprise a controller 103 which may be as shown above in relation to Fig. 1. In some examples the apparatus 101 could comprise one or more filters which may be configured to filter the signal obtained from the broadband detector 209 to remove information indicative of the motion artefacts and leave information relating to the biometric parameter within the signal. The filtered signal could then be processed by the controller 103 to determine the biometric parameter of the subject.

The processing of the signal to determine the biometric parameter of the subject 211 could comprise any suitable processing which enables the motion artefacts to be removed from the signal. In some examples processing the signal to determine a biometric parameter of the subject 211 comprises filtering at least the first and second components of the signal to remove a common mode signal. In such examples the common mode signal comprises motion artefacts which are similar for each of the different components of the signal as a result of the way the respective emitters 203, 205 have been controlled.

In some examples the signal from the broadband detector 209 is filtered at a frequency corresponding to a frequency at which the spectral and spatial distributions of light are alternated. The filtering frequency could be the same as the alternating frequency. The apparatus 101 can therefore provide an output which is indicative of the biometric parameter of the subject.

In some but not necessarily all examples, the system 201 could be connected to other devices and networks. For example the system 201 could be provided in a wearable device which may be configured to communicate with another device such as a computer or mobile phone. In such examples the apparatus 101 is configured to communicate data from the apparatus 101 with or without local storage of the data in a memory 107 at the apparatus 101 and with or without local processing of the data by circuitry or processors at the apparatus 101.

The data may, for example, be measurement data from the detector 209 or data produced by the processing of measurement data from detector 209 by filtering or performing any other process.

The data may be stored in a processed or unprocessed format remotely at one or more devices. The data may be stored at a server.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The apparatus may comprise a communications interface such as, for example, a radio transceiver for communication of data.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train a machine learning network or may be used as a query input to a machine learning network, which provides a response. The machine learning network may for example use linear regression, logistic regression, vector support machines or an acyclic machine learning network such as a single or multi hidden layer neural network.

The processing of the data, whether local or remote, may produce an output. The output may be communicated to the apparatus 101 where it may produce an output sensible to the subject such as an audio output, visual output or haptic output.

Figs. 3A and 3B show another example system 201 and signals that may be obtained using the system 201.

The example system 201 shown in Fig. 3A comprises a broadband detector 209 a plurality of emitters 203, 205 and control means 207. The broadband detector 209 could be coupled to an apparatus 101 which is not shown in Fig. 3A.

The example system 201 of Fig. 3A comprises twelve emitters 203, 205. Six of the emitters 203 are configured to emit a first spectral and spatial distribution of light while the other six emitters 205 are configured to emit a second spectral and spatial distribution of light. It is to be appreciated that other numbers of emitters 203, 205 could be used in other examples of the disclosure.

In the example of Fig. 3A the first emitters 203 configured to emit the first spectral and spatial distribution of light are configured to emit green light. The first emitters 203 may comprise one or more green LEDs. In the example of Fig. 3A the second emitters 205 configured to emit the second spectral and spatial distribution of light are configured to emit red light. The second emitters 205 may comprise one or more red LEDs. It is to be appreciated that other wavelengths of light could be used in other examples of the disclosure.

In the system 201 shown in Fig. 3A the emitters 203, 205 are are positioned surrounding the broadband detector 209. The emitters 203, 205 are arranged in a circular, or substantially circular configuration, so that each of the emitters 203, 205 is an equal, or substantially equal, distance from the broadband detector 209.

The emitters 203, 205 may be configured so that the light from the different emitters 203, 205 has a similar path through a subject 211. This may help to ensure that the different spectral and spatial distributions of light have a similar dependency to the motion artefacts. In the example of Fig. 3A the emitters 203, 205 are arranged in an alternating configuration so that each first emitter 203 is adjacent to two second emitters 205 and similarly each second emitter 205 is adjacent to two first emitters 203. Having the different emitters 203, 205 adjacent to each other causes the different spectral and spatial distributions of light to propagate through similar parts of the subject 211 to help ensure that the motion artifacts will be similar for each of the spectral and spatial distributions of light.

The emitters 203, 205 are coupled to a control means 207 which controls when the emitters 203, 205 are illuminated. In the example of Fig. 3A the control means 207 comprises a signal generator which may be configured to alternate the illumination of the respective emitters 203, 205. The control means 207 may comprise an alternating current signal generator or any other suitable means.

Fig. 3B shows examples of signals generated by the system 201 shown in Fig. 3A. The first signal 301 is the voltage output provided by the generator of the control means 207 as a function of time. In this example the signal 301 is an alternating square wave. The signal 301 provides a positive output voltage followed by a negative output voltage. The pulses of positive output voltage have the same duration as the pulses of negative output voltage.

In the example of Fig. 3B the positive output of the signal generator has a larger magnitude than the negative output. This may cause the different emitters 203, 205 to emit light at different power levels. The different power levels may be selected so that after the light has propagated through the subject 211 the amount of light that is incident on the broadband detector 209 is similar. This may enable the sensitivity of the broadband detector 209 to be selected to detect both the first spectral and spatial distribution of light and the second spectral and spatial distribution of light.

The different power levels may be selected so that the different spectral and spatial distributions of light have a similar dependency to motion artefacts. The respective power levels may be selected so as to facilitate the processing of the output of the broadband detector 209 to remove the common mode signal.

The second signal 303 shows the power output of the second emitters 205 as a function of time. This shows the light that is emitted by the red LEDs. The system 201 is configured so that the red LEDs are activated by the negative components of the control signal 301 from the control means 207. When the control signal 301 is negative the red LEDs emit red light at a selected intensity and when the control signal 301 is positive the red LEDs do not emit any light. The control signal 301 controls the red LEDs in the second emitters 205 so that the red light is either emitted at a set power level or not emitted at all.

The third signal 305 shows the power output of the first emitters 203 as a function of time. This shows the light that is emitted by the green LEDs. The system 201 is configured so that the green LEDs are activated by the positive components of the control signal 301 from the control means 207. When the control signal 301 is positive the green LEDs emit green light at a selected intensity and when the control signal 301 is negative the green LEDs do not emit any light. The control signal 301 controls the green LEDs in the first emitters 203 so that the green light is either emitted at a set power level or not emitted at all.

In the example of Fig. 3B the first emitters 203 are controlled to emit light at a different power level to the second emitters 205. The different power levels may be selected to help to ensure that the different spectral and spatial distributions of light have a similar dependency to motion artefacts as the light is propagated through a subject 211.

The fourth signal 307 shows the intensity of the light that is detected by the broadband detector 209 as a function of time. The broadband detector 209 detects both the red light and the green light. In the example of Figs. 3A and 3B the emission of the different spectral and spatial distributions of light are alternated so that the different spectral and spatial distributions of light are incident on the broadband detector 209 at different times. Therefore intensity of light detected by the broadband detector 209 varies as the different wavelengths of light are incident on the broadband detector 209.

As the broadband detector 209 detects different wavelengths of light the signal 307 provided by the broadband detector 209 comprises two components 311, 313. The first component 311 is provided in response to a first spectral and spatial distribution of light being incident on the broadband detector 209 and the second component 313 is provided in response to a second spectral and spatial distribution of light being incident on the broadband detector 209. In the example shown in Fig. 3B the first spectral and spatial distribution of light is the green light that is emitted by the first emitters 203. This component 311 of the signal 307 will therefore contain information about the motion artefacts and also a biometric parameter such as the heart rate of the subject 211. The second spectral and spatial distribution of light is the red light that is emitted by the second emitters 205. The second emitters 205 are configured so that the red light has a similar dependency to the motion artefacts as the green light, but is less dependent on the biometric parameter. The second component 313 of the signal 307 will therefore contain information about the motion artefacts but not the biometric parameter of the subject 211. The information about the biometric parameter can therefore be obtained by removing the common mode of the different components 311, 313 of the signal 307.

Fig. 4 shows how different spectral distributions of light may be propagated through a subject 211. The example of Fig. 4 shows a first emitter 203 which is configured to emit green light 411, a second emitter 205 which is configured to emit red light and a third emitter 401 which is configured to emit blue light 415. The emitters 203, 205, 401 are positioned adjacent to the skin of a subject 211 so that the different spectral distributions of light can probe the subject 211. Fig. 4 also shows a broadband detector 209 which is configured to detect the different spectral and spatial distributions of light after they have been propagated through the subject 211.

The different wavelengths of light emitted by each of the different emitters 203, 205, 401 propagate to different depths of the tissue of the subject 211. As shown in Fig. 4 the red light 413 is propagated to a deeper depth than the green light 411 and the blue light 415 is propagated to a shallower depth than the green light 411. To ensure that each of the different spectral and spatial distributions of light have a similar dependency to motion artefacts it may be beneficial to position the different emitters 203, 205, 401 so that each of the different spectral and spatial distributions of light propagates through a similar volume of the subject 211 before it is incident on the broadband detector 209.

In the example of Fig. 4 the similar volumes are achieved by positioning the respective emitters 203, 205, 401 at different distances from the broadband detector 209. The relative distances between the emitters 203, 205, 401 and the broadband detector 209 may be dependent on the spectral distribution of light that is emitted by each of the emitters 203, 205, 401. In the example of Fig. 4 the second emitter 205 which emits the red light 413 is positioned closer to the broadband detector 209 than the first emitter 203 which emits the green light 411. Similarly the third emitter 401 which emits the blue light 415 is positioned further away from the broadband detector 209 than the first emitter 203. These positions help to ensure that the red light, 413, green light 411 and blue light 415 all propagate through similar volumes of the subject 211 before they are incident on the broadband detector 209.

Fig. 5 shows an example configuration of a broadband detector 209 and a plurality of emitters 203, 205, 401 which could be used in some implementations of disclosure.

In the example of Fig. 5 the configuration comprises a single broadband detector 209 and eighteen emitters 203, 205, 2410. The eighteen emitters 203, 205, 401 comprise six first emitters 203 configured to emit green light, six second emitters 205 configured to emit red light and six third emitters 401 configured to emit blue light. It is to be appreciated that different numbers of emitters 203, 205, 401 and/or different spectral distributions of light could be used in other examples of the disclosure.

In the example of Fig. 5 the broadband detector 209 is located in the centre of the configuration and the emitters 203, 205, 401 are located around the broadband detector 209. The emitters 203, 205, 401 are located around the broadband detector 209 in a star shaped configuration. The star shaped configuration comprises six stems 501 where each stem 501 comprises a first emitter 203, a second emitter 205 and a third emitter 401. The second emitter 203 is positioned at the end of the stem 501 closest to the broadband detector 209, the first emitter 203 is positioned in the middle of the stem 501 and the third emitter 401 is positioned at the end of the stem 501 furthest away from the broadband detector 209. This positioning ensures that the different spectral distributions of light each propagate through a similar volume of the subject 211 when the emitters 203, 205, 401 and broadband detector 209 are in use.

In the example configuration shown in Fig. 5 the arrangement of the emitters 203, 205, 401 enables the area of the subject 211 surrounding the broadband detector 209 to be probed. This arrangement can smooth out irregularities which could be caused by the inhomogeneity of the subject 211. The irregularities could cause problems if the broadband detector 209 moves relative to the subject 211 during use. This configuration can provide more accurate readings compared with just a single group of emitters as shown in Fig. 4.

Fig. 6 shows another example configuration of a plurality of broadband detectors 209 and a plurality of emitters 203, 205, 401 which could also be used to smooth out irregularities caused by the inhomogeneity of the subject 211 and/or movement of the broadband detectors 209.

In the example of Fig. 6 the configuration comprises three broadband detectors 209 and eighteen emitters 203, 205, 410. The eighteen emitters 203, 205, 401 comprise six first emitters 203 configured to emit green light, six second emitters 205 configured to emit red light and six third emitters 401 configured to emit blue light. It is to be appreciated that different numbers of emitters 203, 205, 401 and/or different spectral distributions of light could be used in other examples of the disclosure.

In the example of Fig. 6 the broadband detectors 209 are provided in a line and two lines of emitters 203, 205, 401 are provided on either side of each of the broadband detectors 209. The lines of emitters 203, 205, 401 are arranged so that the second emitters 205 are closest to the broadband detectors 209 while the third emitters 401 are furthest away.

In the examples of Figs. 5 and 6 a first spectral and spatial distribution of light is used to measure the biometric parameter and two additional spectral and spatial distributions of light are used to monitor the motion artefacts. In the examples shown the first spectral and spatial distribution of light comprises green light while the other spectral and spatial distributions of light comprise light that is not green. In these examples the light that is not green comprises red light and blue light.

In these examples the additional spectral and spatial distributions of light may be selected so that one of the spectral and spatial distributions of light has a shorter wavelength than the first spectral and spatial distribution of light and the other spectral and spatial distribution has a longer wavelength of light. This may enable differences in the way the spectral and spatial distributions depend on the motion artefacts to be cancelled out. For instance, if the motion artefacts have a greater effect on the longer wavelengths of light it may have a lesser effect on the shorter wavelengths of light. The respective wavelengths of the light may be selected so that these effects are cancelled out, or substantially cancelled out.

In other examples other wavelengths of light could be used instead. For instance instead of using red and blue light, white light could be used. The white light comprises a range of different wavelengths some of which may show similar dependencies on the motion artefacts to the green light while having a different dependency on the biometric parameter.

Fig. 7 shows a filter arrangement 701 that may be used to filter the output of the broadband detector 209 in some examples of the disclosure. The filter arrangement 701 may be used to filter at least the first and second components of the output signal to separate a common mode signal from a differential signal.

In the example of Fig. 7 the filter arrangement 701 comprises a bias tee 703. The bias tee 703 is coupled to the broadband detector 209 and comprises a capacitor 705 and an inductor 707. The capacitor 705 is configured to allow the differential signal through but block the common mode signal. The inductor 707 is configured to allow the common mode signal through but blocks the differential signal. In the example of Fig. 7 the differential signal comprises the alternating current part of the output of the broadband detector 209. This may comprise information about the biometric parameter of the subject 211. The common mode signal is direct current part of the output of the broadband detector 209. This may comprise information about the motion artefacts which are common to all of the components of the signal provided by the broadband detector 209.

The filter arrangement 701 also comprises a band pass filter 709 which is configured to remove noise from the differential signal. The bandpass filter 709 could be configured to filter the differential signal at a frequency which corresponds to the frequency at which the spectral and spatial distributions of light are alternated. That is, the frequencies which are removed by the filter comprise the frequency at which the different emitters 203, 205, 401 are switched on and off by the control signal.

The filter arrangement may also comprise amplifiers 711 which are configured to amplify the filtered signals. In the example of Fig. 7 the amplifiers 711 comprise transimpedance amplifiers which may be configured to convert current to voltage. Other types of amplifiers 711 could be used in other examples of the disclosure.

Fig. 8 shows example signals 305, 303, 307 that could be used in some examples of the disclosure.

The example signal 305 shows the light that is emitted by a first emitter 203 as a function of time and the example signal 303 shows the light that is emitted by a second emitter 205 as a function of time. These signals may be similar to the signals shown in Fig. 3B. In the example of Fig. 8 the light that is emitted by the first emitter 203 comprises green light and the light that is emitted by the second emitter 205 comprises red light.

The first emitter 203 and the second emitter 205 are alternated so that the light emitted is in antiphase. That is when the first emitter 203 is emitting light the second emitter 205 is not and when the second emitter 205 is emitting light the first emitter 203 is not. The frequency at which the emitters 203, 205 are alternated may be much larger than the frequency of the biometric parameter that is to be measured. For instance, in the example of Fig. 8 the time scale shown may be several milliseconds. The frequency of alternation may be much greater than the frequency of the heart rate of the subject 211, or any other suitable parameter. This enables a filter to be selected which can remove noise form the signals and separate the components of the signals without removing information about the biometric parameter.

The third signal 307 comprises the output signal that is provided by the broadband detector 209. This may be similar to the signal shown in Fig. 3B and comprises a first component 311 corresponding to the first spectral and spatial distribution of light and a second component 313 corresponding to the second spectral and spatial distribution of light. These components 311, 313 are detected at different times.

The signal 307 comprises a common mode signal 803. The common mode signal 803 is the part that is common to both the first component 311 and the second component 313. As the spectral and spatial distributions of light are selected to have a similar dependency to motion artifacts the common mode signal 801 relates to the motion artefacts. The signal 308 also comprises a differential signal 801. The differential signal is the part that is different between the first component 311 and the second components 313. As the spectral and spatial distributions of light are selected to have a different dependency to the biometric parameter the differential signal 801 relates to the biometric parameter. By processing the signal 307 to separate the common mode signal and the differential signal the information about the biometric parameter can be extracted.

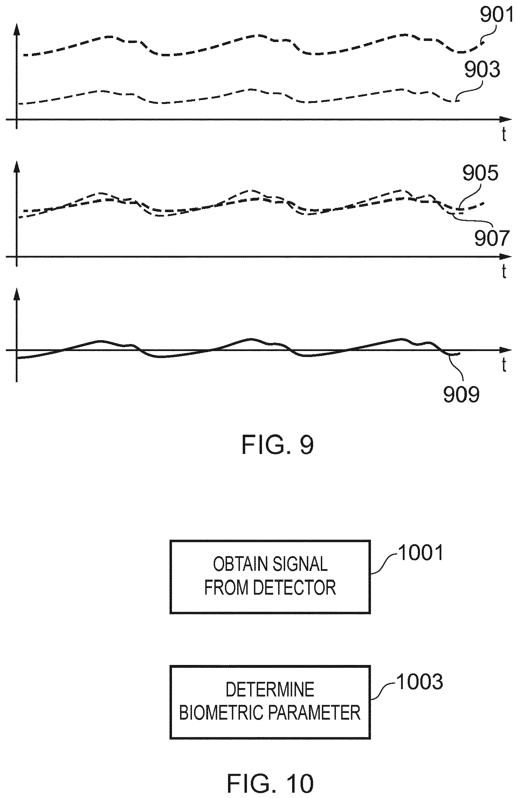

Fig. 9 shows more example output signals of a broadband detector 209 that could be used in examples of the disclosure. The signals shown in Fig. 9 are obtained over a longer period of time than that shown in Fig. 8. This enables features of the biometric parameter to be seen in Fig. 9. For instance the signals shown in Fig. 8 may be obtained over a period of milliseconds while the signals shown in Fig. 9 may be obtained over a period of seconds.

Signal 901 may be the first component 311 of the signal provided by the broadband detector 209 and signal 903 may be the second component of the signal provided by the broadband detector 209. In this example the signals 901, 903 are shown as dashed lines to indicate that the components are detected alternately.

Signals 905 and 907 correspond to signals 901 and 903 after they have been adjusted for the power differences in the different emitters 203, 205. In some examples it might not be needed to adjust the power levels of the received signal as the power levels of the emitted light could be adjusted instead.

Signal 909 shows the differential signal which is obtained after the common mode signal has been removed. The differential signal may provide information about a biometric parameter such as the heart rate of the subject 211.

In the examples of Figs. 8 and 9 the signals are obtained for a first spectral and spatial distribution of light and a second spectral and spatial distribution of light. In other examples the signals could be obtained for more than two spectral and spatial distributions of light. In such examples the light could be emitted so that each spectral and spatial distribution of light is detected at a different point in time. In other examples the spectral and spatial distributions of light that are dependent upon the biometric parameter could be emitted at a different time to the spectral and spatial distributions of light that are not dependent upon the biometric parameter. For instance the red and blue light could be emitted simultaneously but could be emitted at a different time to the green light.

In some examples the additional spectral and spatial distributions of light could be used to measure a different biometric parameter to the first spectral and spatial distribution of light. For instance a third spectral and spatial distribution of light could use light with wavelength within the near infrared range of the spectrum and this could be used to measure blood oxygenation levels while the first spectral and spatial distribution of light is used to measure heart rate. Different wavelengths of light and different biometric parameters could be used in other examples of the disclosure.

Fig. 10 shows example method that could be implemented using the apparatus 101 and systems 201 as shown above.

The method comprises, at block 1001, obtaining a signal from a broadband detector 209 in response to light that has been propagated through a subject 211 being incident on the broadband detector 209. The signal that is obtained from the broadband detector 209 comprises a first component and a second component where the first component is provided in response to a first spectral and spatial distribution of light being incident on the broadband detector 209 and the second component is provided in response to a second spectral and spatial distribution of light being incident on the broadband detector 209. An emitter 203 of the first spectral and spatial distribution of light and an emitter 205 of the second spectral and spatial distribution of light are controlled so that the first spectral and spatial distribution of light and the second spectral and spatial distribution light have a similar dependency to motion artefacts but a different dependency to a biometric parameter.

The method also comprises, at block 1003, processing the components of the obtained signal to enable the biometric parameter of the subject 211 to be determined. The processing may comprises filtering the signal to separate a common mode signal from a differential signal. The biometric parameter can then be determined from the differential signal.

The examples of the disclosure therefore provide the technical effect of enabling motion artefacts to be removed from a signal, such as a photoplethsmography signal. This provides for improved accuracy in the measurement of the heart rate or other suitable biometric parameter.

Examples of the disclosure also use only a single broadband detector 209 to detect multiple different wavelengths of light. This can provide for a simple and cost effective method of detecting photoplethsmography signals compared to systems which use a plurality of different detectors which are sensitive to different spectral distributions of light. In examples of the disclosure the broadband detector 209 provides a single output with a plurality of different components which may be processed using a simple filter arrangement in order to obtain the differential signal.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to 'comprising only one...' or by using 'consisting'.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although embodiments have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain embodiments, those features may also be present in other embodiments whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer and exclusive meaning.

The presence of a feature (or combination of features) in a claim is a reference to that feature) or combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:
obtaining a signal from a broadband detector in response to light that has been propagated through a subject being incident on the broadband detector, wherein the signal comprises at least a first component and a second component where the first component is provided in response to a first spectral and spatial distribution of light being incident on the broadband detector and the second component is provided in response to at least one other spectral and spatial distribution of light being incident on the broadband detector and wherein an emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to a biometric parameter; and
processing the first component and the second component of the obtained signal to enable the biometric parameter of the subject to be determined.

2. An apparatus as claimed in claim 1 wherein controlling the emitters so that the first spectral and spatial distribution of light and the at least one other spectral and spatial distribution of light have a similar dependency to motion artefacts but a different dependency to the biometric parameter comprises selecting one or more wavelengths of light to use in the spectral distributions.

3. An apparatus as claimed in any preceding claim wherein controlling the emitters so that the first spectral and spatial distribution of light and the at least one other spectral and spatial distribution light have a similar dependency to motion artefacts but a different dependency to the biometric parameter comprises positioning the emitters relative to the broadband detector.

4. An apparatus as claimed in any preceding claim wherein the signal also comprises at least a second component and a third component wherein the second component is provided in response to a second spectral and spatial distribution of light being incident on the broadband detector and the third component is provided in response to a third spectral and spatial distribution of light being incident on the broadband detector.

5. An apparatus as claimed in any preceding claim wherein the emission of the respective spectral and spatial distributions of light are controlled so that the respective spectral and spatial distributions of light are incident on the broadband detector at different times.

6. An apparatus as claimed in any preceding claim wherein the first spectral and spatial distribution of light comprises green light.

7. An apparatus as claimed in claim 6 wherein the at least one other spectral and spatial distribution of light comprise at least one of; red light, blue light.

8. An apparatus as claimed in any preceding claim wherein the biometric parameter comprises the heart rate of the subject.

9. An apparatus as claimed in any preceding claim wherein processing the respective components of the signal to determine a biometric parameter of the subject comprises filtering at least the respective components of the signal to separate a common mode signal from a differential signal.

10. An apparatus as claimed in claim 9 wherein the differential signal comprises information about the biometric parameter.

11. An apparatus as claimed in any of claims 9 to 10 wherein the respective components of the signal are filtered at a frequency corresponding to a frequency at which the spectral and spatial distributions of light are alternated.

12. An apparatus as claimed in any preceding claim wherein the emitter of the first spectral and spatial distribution of light and the emitter of the at least one other spectral and spatial distribution of light are controlled so that a similar power level is incident on the broadband detector for both of the spectral and spatial distributions of light.

13. A method comprising:
obtaining a signal from a broadband detector in response to light that has been propagated through a subject being incident on the broadband detector, wherein the signal comprises at least a first component and a second component where the first component is provided in response to a first spectral and spatial distribution of light being incident on the broadband detector and the second component is provided in response to at least one other spectral and spatial distribution of light being incident on the broadband detector and wherein an emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to a biometric parameter; and
processing the first component and the second component of the obtained signal to enable the biometric parameter of the subject to be determined.

14. A computer program comprising computer program instructions that, when executed by processing circuitry, cause:
obtaining a signal from a broadband detector in response to light that has been propagated through a subject being incident on the broadband detector, wherein the signal comprises at least a first component and a second component where the first component is provided in response to a first spectral and spatial distribution of light being incident on the broadband detector and the second component is provided in response to at least one other spectral and spatial distribution of light being incident on the broadband detector and wherein an emitter of the first spectral and spatial distribution of light and an emitter of the at least one other spectral and spatial distribution of light are controlled so that the first spectral and spatial distribution of light and the at least one other spectral and spatial distribution light have a similar dependency to motion artefacts but a different dependency to a biometric parameter; and
processing the first component and the second component of the obtained signal to enable the biometric parameter of the subject to be determined.

15. A system comprising:
at least a first emitter configured to emit light of a first spectral and spatial distribution;
at least a second emitter configured to emit light of a second spectral and spatial distribution;
at least one broadband detector configured to detect at least the first spectral and spatial distribution of light and the second spectral and spatial distribution of light after the respective spectral and spatial distributions of light have propagated through a subject such that the detected signals enable a biometric parameter of the subject to be determined; and
control means configured to control the emission of the first spectral and spatial distribution of light and the second spectral and spatial distribution of light so that they are incident on the broadband detector at different times;
wherein the first emitter and the second emitter are controlled so that the first spectral and spatial distribution of light and the second spectral and spatial distribution of light have a similar dependency to motion artefacts but a different dependency to the biometric parameter.
